# FASCICULE DE BREVET EUROPEEN

(11) **EP 0 556 096 B1**
(45) Date de publication et mention de la délivrance du brevet: **16.09.1998**
(21) Numéro de dépôt: 93400279.1
(22) Date de dépôt: 04.02.1993
(51) Int. Cl.: A61L 2/04, A61K 35/16

(54) **Composition pour stabiliser le plasma sanguin en cours de pasteurisation et procédé de pasteurisation comprenant l'utilisation de cette composition**
Zusammensetzung zur Stabilisierung von Blutplasma im Laufe der Pasteurisation und Pasteurisationsverfahren unter Verwendung dieser Zusammensetzung
Composition for stabilizing blood plasma during pasteurization and pasteurization process comprising the use of said composition

(30) Priorité: 13.02.1992 FR 9201604
(43) Date de publication de la demande: 18.08.1993
(73) Titulaire: ASSOCIATION POUR L'ESSOR DE LA TRANSFUSION SANGUINE DANS LA REGION DU NORD, F-59012 Lille (FR)
(72) Inventeur: Burnouf, Miryana (née Radosevich), F-59136 Wavrin (FR)
(74) Mandataire: Lhuillier, René

(56) Documents cités:
- EP-A- 0 053 338
- EP-A- 0 103 196
- EP-A- 0 106 269
- EP-A- 0 117 064
- EP-A- 0 139 975
- WO-A-91/01762
- DE-A- 3 400 413

## Description

L'invention concerne une composition permettant de stabiliser le plasma sanguin en cours de pasteurisation, un procédé utilisant ladite composition et les solutions plasmatiques ainsi obtenues, particulièrement destinées aux thérapies de remplissage en plasma ou aux thérapies de substitution en facteurs de la coagulation sanguine.

Le plasma humain total ou dépourvu de cryoprotéines est encore utilisé comme "thérapie de remplissage" pour les grands brûlés, les sujets gravement traumatisés ou ayant subi d'importantes interventions chirurgicales, c'est-à-dire dans tous les cas où les patients subissent d'importantes pertes de fluides.

On utilise généralement, pour ce type de thérapie, du plasma provenant de dons unitaires, de sujets sains et préalablement contrôlés, pour éviter les risques de transmission de maladies virales. Cette procédure ne permet cependant pas d'éliminer tous les risques de contamination par des virus en phase pré-sérologique, en particulier les différents virus de l'hépatite et du SIDA.

Il apparaît donc toujours important de poursuivre la mise au point de méthodes d'inactivation virale et de compositions stabilisantes qui préservent les fonctions biologiques essentielles du plasma.

Il a été démontré que le virus de l'Hépatite B était totalement inactivé par un chauffage à 60°C pendant 10 heures, en présence de citrate de sodium 0,5 M (Tabor et al. Thrombosis Res. 22, 1981, 233-238). Toutefois ce traitement entraîne une perte d'activité biologique de certaines protéines du plasma (Tabor et al. et Barrowcliffe et al. Fr. J. Haematology 55, 1983, 37-46).

Différentes protéines d'intérêt thérapeutique, purifiées à partir du plasma sanguin, ont pu être soumises à une pasteurisation classique à 60°C pendant 10 heures en présence de divers stabilisants. On constate toutefois que des compositions qui stabilisent une activité biologique peuvent être totalement inefficaces sur une autre.

Ces différentes données expérimentales ont conduit à l'idée que l'inactivation virale du plasma total (plasma frais ou plasma frais congelé) ou du plasma dépourvu de cryoprotéines ne pouvait pas être réalisée par une pasteurisation.

Toutefois le besoin médical existant pour du plasma total, la Demanderesse a cherché à mettre au point des compositions assurant la protection simultanée de l'activité biologique de tous les facteurs d'intérêt thérapeutique du plasma contre la dénaturation en cours de pasteurisation.

Ainsi, dans la demande de brevet WO 92.00767 la Demanderesse a montré qu'un mélange de sorbitol, d'héparine, de lysine et de CaCl₂ stabilisait efficacement l'activité biologique des facteurs plasmatiques au cours du procédé de pasteurisation.

Cependant la présence d'héparine dans ce mélange stabilisant et donc dans la préparation finale ne permet pas d'injecter ce plasma à des patients déjà soumis à un traitement à l'héparine.

C'est pourquoi la Demanderesse a cherché une autre composition remplissant la même fonction de stabilisation.

Ayant observé précédemment que le sorbitol offrait une certaine protection contre la dénaturation thermique, mais avec des résultats variables d'un échantillon de plasma à l'autre et avec de faibles rendements, la Demanderesse a cherché différents additifs dont le mélange avec le sorbitol augmenterait son pouvoir stabilisant, et, en particulier, elle a obtenu de bons résultats par addition d'une quantité de saccharose voisine de 50 % de la quantité de sorbitol.

D'autre part, l'observation d'une instabilité du CaCl₂ au cours du chauffage prolongé a conduit à le remplacer par du gluconate de calcium qui résiste bien aux conditions de pasteurisation.

En outre, l'addition de citrate trisodique a amélioré l'effet protecteur du mélange, à condition d'en ajuster soigneusement la concentration, en effet, une dose trop faible permet la coagulation spontanée du plasma et une dose trop forte ralentit l'activité ultérieure des facteurs de coagulation au cours de l'utilisation thérapeutique du plasma.

Enfin le mélange est additionné d'au moins deux acides aminés, de préférence la lysine et l'arginine.

Ainsi la composition stabilisante selon l'invention est constituée d'un mélange de sorbitol, de saccharose, de gluconate de calcium, de citrate trisodique, de lysine et d'arginine. Les concentrations suivantes des différents additifs sont ajustées pour un litre de plasma initial à pasteuriser :
- sorbitol, de 800 à 1.400 g et de préférence 1.300 g
- saccharose, de 400 à 600 g et de préférence 514 g
- gluconate de calcium,de 3 à 5 mM et de préférence 4 mM
- citrate trisodique, de 8 à 25 mM et de préférence 15 mM
- lysine, de 1 à 10 g et de préférence 5 g
- arginine, de 1 à 10 g et de préférence 5 g.

La composition selon l'invention est additionnée au plasma frais ou au plasma frais congelé-décongelé ou au plasma dépourvu des protéines du cryoprécipité, avant de soumettre celui-ci à une pasteurisation par chauffage à 60°C±1°C, à l'état liquide, pendant 10 heures.

Après la pasteurisation, la température est abaissée progressivement jusqu'à 20°C et la solution est soumise à une dialyse pour éliminer le sorbitol et le saccharose. Le tampon de dialyse est à pH 7,5 et contient du citrate trisodique 10 mM, du gluconate de calcium 4 mM, du chlorure de sodium 0,1 M, de la lysine à une concentration de 10 g/l et de l'arginine à une concentration de 3 g/l. Des tampons de dialyse de composition différente peuvent aussi être utilisés en fonction des besoins. La solution est ensuite concentrée pour rétablir le dosage physiologique des protéines plasmatiques. La solution résultante est soumise à une filtration clarifiante puis stérilisante, conditionnée puis congelée ou lyophilisée.

L'invention concerne également le procédé de pasteurisation du plasma qui comprend l'addition de la composition selon l'invention dans le plasma, avant son chauffage, et ensuite la dialyse du plasma pasteurisé contre le tampon tel que défini plus haut.

Ce procédé est particulièrement avantageux parce qu'il peut être appliqué à de grands lots de plasma provenant de plusieurs récoltes indépendantes. Ainsi la pasteurisation de lots de l'ordre de 60 litres ou plus permet, après avoir effectué les contrôles appropriés, de garantir une constance de qualité biochimique du produit.

L'invention concerne également les solutions plasmatiques pasteurisées obtenues par le procédé selon l'invention, lesdites solutions comprenant le plasma total ou le plasma dépourvu de certaines protéines (comme les protéines du cryoprécipité) et destinées à un usage thérapeutique, soit pour le remplacement du plasma total, soit pour compenser une déficience en un facteur sanguin particulier.

La composition et le procédé selon l'invention peuvent également être appliqués à du plasma d'origine animale. Ils seront, par exemple, avantageusement appliqués au sérum foetal bovin utilisé comme additif pour les cultures cellulaires plus particulièrement destinées à la préparation de produits thérapeutiques.

L'exemple suivant décrit un mode de réalisation de l'invention sans toutefois en limiter la portée.

### Exemple

Soixante litres de plasma décongelé sont additionnés du mélange suivant, sous agitation douce :

| | quantité par litre de plasma initiale |
|---|---|
| sorbitol | 1.300 g |
| saccharose | 514 g |
| gluconate de calcium | 4 mM |
| citrate trisodique | 15 mM |
| lysine | 5 g |
| arginine | 5 g |

La pasteurisation est réalisée dans une cuve thermostatée, par chauffage à 60°C pendant 10 heures.

Après la pasteurisation, la température est abaissée progressivement jusqu'à 20°C puis la solution est soumise à une dialyse pour éliminer le sorbitol et le saccharose.

La dialyse est effectuée, par exemple, sur un système d'ultrafiltration sur cassettes (Pellicon 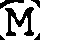 ).

La composition du tampon de dialyse est la suivante :

| | |
|---|---|
| - citrate trisodique | 10 mM |
| - lysine | 10 g/l |
| - arginine | 3 g/l |
| - gluconate de calcium | 4 mM |
| - chlorure de sodium | 0,1 M |

L'élimination du sorbitol est vérifiée par dosage colorimétrique et celle du saccharose par dosage enzymatique, sur le produit fini.

La dialyse peut être suivie, le cas échéant, d'une concentration sur le même matériel pour ramener les taux en facteurs de coagulation à près d'1 U/ml comme dans un plasma thérapeutique de bonne qualité. Le produit est dialysé à une osmolarité de 370 mosmol/l et un pH de 7 - 7,5.

Le produit est ensuite stérilisé par filtration, par exemple sur filtre Millipack 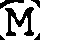 40 (Millipore 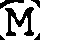 ) à pores de 0,22 µm. Puis la répartition du matériel s'effectue dans des poches de plastique ou des flacons pour congélation ou pour lyophilisation.

L'efficacité de la pasteurisation sur l'inactivation virale a été évaluée sur un choix de virus recommandé par le "groupe d'experts en biotechnologie/pharmacie des instances européennes pour la validation des procédés d'élimination et d'inactivation virale des produits biologiques". Les résultats sont présentés dans le tableau suivant :

| VIRUS | GENOME | STRUCTURE | REDUCTION D'INFECTIOSITE (log 10) | DUREE |
|---|---|---|---|---|
| VIH | ARN | E | >5 | <10 min |
| POLIO SABIN 1 | ARN | NE | >6,23 | <5 heures |
| VACCINE | ADN | E | >4,30 | <1 heure |
| HERPES D'AUJESZKY | ADN | E | >4,15 | >5 heures |
| PARAINFLUENZA TYPE 3 | ARN | E | >6,30 | <2 heures |
| REOVIRUS TYPE 3 | ARN | NE | >3,28 | ≤10 heures |
| SINDBIS | ARN | E | >5,74 | <5 heures |
| E = enveloppé NE= non enveloppé | | | | |

Les résultats des contrôles de qualité effectués sur 6 lots pilotes consécutifs sont indiqués dans les tableaux suivants.

**Tableau II**

| Récupération des facteurs sanguins | | |
|---|---|---|
| | CONCENTRATION (U/ml) | RENDEMENT (%) |
| Protéines (g/l) | 60,0 ± 106 | 93 ± 2 |
| Facteur VIII : c (1 temps) (UI/ml) | 1,10 ± 0,26 | 94 ± 7 |
| Facteur VIII : c (chromogénique) | 0,70 ± 0,09 | 83 ± 13 |
| Facteur V : c | 1,12 ± 0,25 | 83 ± 4 |
| Facteur XI : c | 0,98 ± 0,06 | 88 ± 7 |
| Facteur IX : c | 0,64 ± 0,10 | 59 ± 7 |
| Facteur VII : c | 0,65 ± 0,05 | 67 ± 6 |
| Fibrinogène Ag (g/l) | 3,10 ± 0,28 | 92 ± 5 |
| Fibrinogène coagulable (g/l) | 2,50 ± 0,17 | 92 ± 3 |
| Activité antithrombine III (g/l) | 0,99 ± 0,04 | 92 ± 3 |
| Activité alpha-1-antitrypsine (g/l) | 0,96 ± 0,10 | 92 ± 3 |
| APTT | 35-38 secondes | - |

**Tableau III**

| Contrôle de qualité : absence de protéases et de produits de dégradation. | |
|---|---|
| | CONCENTRATION (U/ml) |
| Thrombine | <0,01 |
| PKA (%) | <2 |
| Kallicréine (%) | 3 |
| Facteur IXa (U/ml) | <0,1 |
| Facteur Xa (U/ml) | <0,1 |
| Facteur VIIb/FVIIa | 0,97 |
| Activité protéolytique (S.2288 - Δ DO/mn) | 0,03 |
| Produits de dégradation du fibrinogène (ng/ml) | <250 |

**Tableau IV**

| Contrôle de qualité sur modèles animaux, in vivo. | |
|---|---|
| Tests sur rats: | |
| - tension artérielle | < - 2 % |
| - rythme cardiaque | inchangé |
| - toxicité (injection intraveineuse -25ml/kg) | absence de toxicité |

| Tests sur lapins : | |
|---|---|
| - pyrogénicité | non pyrogène |

Le plasma pasteurisé en présence de la composition stabilisante définie plus haut répond aux normes prévues pour une utilisation thérapeutique.

## Revendications

1. Composition pour stabiliser le plasma sanguin en cours de pasteurisation, caractérisée en ce qu'elle est constituée d'un mélange de sorbitol, de saccharose, de gluconate de calcium, de citrate trisodique, de lysine et d'arginine.

2. Composition selon la revendication 1, caractérisée en ce que la concentration de sorbitol est comprise entre 800 et 1.400 g/litre de plasma initial.

3. Composition selon la revendication 2, caractérisée en ce que la concentration de sorbitol est de 1.300 g/l.

4. Composition selon l'une quelconque des revendications 1 à 3, caractérisée en ce que la concentration de saccharose est comprise entre 400 et 600 g/l.

5. Composition selon la revendication 4, caractérisée en ce que la concentration de saccharose est de 514 g/l.

6. Composition selon l'une quelconque des revendications 1 à 5, caractérisée en ce que la concentration de gluconate de calcium est comprise entre 3 et 5 mM.

7. Composition selon la revendication 6, caractérisée en ce que la concentration de gluconate de calcium est de 4 mM.

8. Composition selon l'une quelconque des revendications 1 à 7, caractérisée en ce que la concentration de citrate trisodique est comprise entre 8 et 25 mM.

9. Composition selon la revendication 8, caractérisée en ce que la concentration de citrate trisodique est de 15 mM.

10. Composition selon l'une quelconque des revendications 1 à 9, caractérisée en ce que les concentrations de lysine et d'arginine sont comprises entre 1 et 10 g/l.

11. Composition selon la revendication 10, caractérisée en ce que les concentrations de lysine et d'arginine sont de 5 g/l.

12. Procédé de pasteurisation du plasma sanguin caractérisé en ce qu'il comprend l'addition de la composition selon l'une quelconque des revendications 1 à 11 dans le plasma, avant l'étape de chauffage et, ensuite, l'élimination des sucres de ladite composition, après l'étape de chauffage, par dialyse contre du tampon contenant du citrate trisodique, du gluconate de calcium, du chlorure de sodium, de la lysine et de l'arginine.

13. Procédé selon la revendication 12, caractérisé en ce que le tampon de dialyse contient du citrate trisodique 10 mM, du gluconate de calcium 4 mM, du chlorure de sodium 0,1 M, 10 g/l de lysine et 3 g/l d'arginine.

14. Procédé selon la revendication 12 ou 13, caractérisé en ce qu'il est applicable à des volumes de plasma compris entre 1 litre et plusieurs centaines de litres.

## Claims

1. Composition for stabilizing blood plasma during pasteurization, characterized in that it is composed of a mixure of sorbitol, saccharose, calcium gluconate, trisodium citrate, lysine and arginine.

2. Composition according to claim 1, characterized in that the sorbitol concentration is between 800 and 1,400 g/litre of initial plasma.

3. Composition according to claim 2, characterized in that the sorbitol concentration is 1,300 g/l.

4. Composition according to any one of claims 1 to 3, characterized in that the saccharose concentration is beteen 400 and 600 g/l.

5. Composition according to claim 4, characterized in that the saccharose concentration is 514 g/l.

6. Composition according to any one of claims 1 to 5, characterized in that the calcium gluconate concentration is between 3 and 5 mM.

7. Composition according to claim 6, characterized in that the calcium gluconate concentration is 4 mM.

8. Composition according to any one of claims 1 to 7, characterized in that the trisodium citrate concentration is between 8 and 25 mM.

9. Composition according to claim 8, characterized in that the trisodium citrate concentration is 15 mM.

10. Composition according to any one of claims 1 to 9, characterized in that the lysine and arginine concentrations are between 1 and 10 g/l.

11. Composition according to claim 10, characterized in that the lysine and arginine concentrations are 5 g/l.

12. Process for pasteurizing blood plasma, characterized in that it includes the addition to the plasma of the composition according to any one of claims 1 to 11 prior to the heating step, and then removal of the sugars from the said composition, after the heating step, by dialysis against a buffer containing trisodium citrate, calcium gluconate, sodium chloride, lysine and arginine.

13. Process according to claim 12, characterized in that the dialysis buffer contains 10mM of trisodium citrate, 4 mM of calcium gluconate, 0.1 M of sodium chloride, 10 g/l of lysine and 3 g/l of arginine.

14. Process according to claim 12 or 13, characterized in that it is applicable to volumes of plasma of between 1 litre and several hundreds of litres.

## Patentansprüche

1. Zusammensetzung zur Stabilisierung des Blutplasmas bei der Pasteurisierung, dadurch gekennzeichnet, daß sie aus einer Mischung von Sorbit, Saccharose, Calciumgluconat, Trinatriumcitrat, Lysin und Arginin besteht.

2. Zusammensetzung nach Anspruch 1, dadurch gekennzeichnet, daß die Sorbit-Konzentration zwischen 800 und 1400 g pro Liter des ursprünglichen Plasmas liegt.

3. Zusammensetzung nach Anspruch 2, dadurch gekennzeichnet, daß die Sorbit-Konzentration 1300 g/l beträgt.

4. Zusammensetzung nach einem der Ansprüche 1 bis 3, dadurch gekennzeichnet, daß die Saccharose-Konzentration zwischen 400 und 600 g/l liegt.

5. Zusammensetzung nach Anspruch 4, dadurch gekennzeichnet, daß die Saccharose-Konzentration 514 g/l beträgt.

6. Zusammensetzung nach einem der Ansprüche 1 bis 5, dadurch gekennzeichnet, daß die Calciumgluconat-Konzentration zwischen 3 und 5 mmol/l liegt.

7. Zusammensetzung nach Anspruch 6, dadurch gekennzeichnet, daß die Calciumgluconat-Konzentration 4 mmol/l beträgt.

8. Zusammensetzung nach einem der Ansprüche 1 bis 7, dadurch gekennzeichnet, daß die Trinatriumcitrat-Konzentration zwischen 8 und 25 mmol/l liegt.

9. Zusammensetzung nach Anspruch 8, dadurch gekennzeichnet, daß die Trinatriumcitrat-Konzentration 15 mmol/l beträgt.

10. Zusammensetzung nach einem der Ansprüche 1 bis 9, dadurch gekennzeichnet, daß die Lysin- und Arginin-Konzentrationen zwischen 1 und 10 g/l liegen.

11. Zusammensetzung nach einem der Ansprüche 1 bis 9, dadurch gekennzeichnet, daß die Lysin- und Arginin-Konzentrationen 5 g/l betragen.

12. Verfahren zur Pasteurisierung des Blutplasmas, dadurch gekennzeichnet, daß es vor dem Schritt der Erwärmung die Zugabe der Zusammensetzung gemäß einem der Ansprüche 1 bis 11 zum Plasma und dann nach dem Schritt der Erwärmung die Entfernung der Zucker der genannten Zusammensetzung durch Dialyse gegen einen Trinatriumcitrat, Calciumgluconat, Natriumchlorid, Lysin und Arginin enthaltenden Puffer umfaßt.

13. Verfahren nach Anspruch 12, dadurch gekennzeichnet, daß der Puffer für die Dialyse 10 mmol/l Trinatriumcitrat, 4 mmol/l Calciumgluconat, 0,1 mol/l Natriumchlorid, 10 g/l Lysin und 3 g/l Arginin enthält.

14. Verfahren nach Anspruch 12 oder 13, dadurch gekennzeichnet, daß es auf Plasmavolumina anwendbar ist, die zwischen einem Liter und mehreren hundert Litern liegen.
